# EUROPÄISCHE PATENTSCHRIFT

(11) **EP 3 512 434 B1**
(45) Veröffentlichungstag und Bekanntmachung des Hinweises auf die Patenterteilung: **30.10.2024**
(21) Anmeldenummer: 17772642.9
(22) Anmeldetag: 15.09.2017
(51) Int. Cl.: A61B 10/02

(54) **BIOPSIEPISTOLE**
BIOPSY GUN
PISTOLET DE BIOPSIE

(30) Priorität: 16.09.2016 DE 202016105165 U
(43) Veröffentlichungstag der Anmeldung: 24.07.2019
(60) Teilanmeldung: 21211589.3
(73) Patentinhaber: Peter Pflugbeil GmbH, 85604 Zorneding (DE)
(72) Erfinder: KNEISSL, Florian, 80639 München (DE)
(74) Vertreter: TBK
(86) Internationale Anmeldenummer: PCT/EP2017/073242
(87) Internationale Veröffentlichungsnummer: WO 2018/050805

(56) Entgegenhaltungen:
- DE-A1- 102008 038 414
- DE-U1- 202011 004 277
- US-A- 5 842 999
- US-A1- 2004 097 830
- US-A1- 2004 097 832
- US-A1- 2004 158 172
- US-A1- 2012 078 133
- US-A1- 2015 105 690
- US-A1- 2015 148 704

## Beschreibung

Aus dem Stand der Technik ist eine handspannbare Biopsiepistole bekannt, bei der eine Kanüle und eine in der Kanüle geführtes Stilett mittels zwei aufeinanderfolgenden Hüben eines Spanngriffs gespannt werden können. Zu diesem Zweck ist die Kanüle an einem Kanülenschlitten befestigt und das Stilett ist an einem Stilettschlitten befestigt, und beide Schlitten sind in einem Gehäuse der Biopsiepistole nebeneinander angeordnet und in der Längsrichtung eines Gehäuses der Biopsiepistole in dem Gehäuse verschiebbar gelagert. Der Spanngriff hat ein längliches Element, das drehfest an dem Spanngriff angebracht ist und seitlich gebogen werden kann. Am vorderen Ende des länglichen Elements sind seitlich zwei Zähne ausgebildet, die in entsprechende Vertiefungen des Kanülenschlittens bzw. des Stilettschlittens eingreifen können.

Im entspannten Zustand von Kanülenschlitten und Stilettschlitten ist der entsprechenden Zahn des länglichen Elements mit der Vertiefung des Kanülenschlittens in Eingriff, sodass während des Spannens des Kanülenschlittens das längliche Element in Querrichtung gebogen wird, damit der der Vertiefung des Stilettschlittens entsprechende Zahn an der Vertiefung des Stilettschlittens vorbeigeführt werden kann und im ersten Hub nur der Kanülenschlitten, nicht aber der Stilettschlitten vorgespannt wird.

Nachdem der Kanülenschlitten vorgespannt wurde und der Spanngriff auf seine entspannte Ausgangsposition zurückkehrt, stellt sich auch der verbogene Zustand des länglichen Elements zurück, sodass im zweiten Hub der andere Zahn, der der Vertiefung des Stilettschlittens entspricht, mit dieser in Eingriff gelangt und somit der Stilettschlitten vorgespannt werden kann.

Damit das längliche Element in Querrichtung leicht genug gebogen werden kann, ist es erforderlich, dass es eine Mindestlänge hat. Dies führt dazu, dass die gesamte Biopsiepistole eine Mindestlänge hat, die es erschweren kann, die Biopsiepistole auf bequeme Weise einhändig vorzuspannen.

US 2004/158172 A1 zeigt ein Gewebeentnahmegerät zur Verwendung mit einer Innennadel und einer Außenkanüle. Die Vorrichtung umfasst einen verschiebbar angeordneten, federvorgespannten Träger für die Innennadel und die Kanüle. Ein Spannmechanismus ist betreibbar, um nacheinander den ersten Träger in seine gespannte Position und den zweiten Träger in seine gespannte Position zu bewegen. Der Spannmechanismus umfasst einen manuell betätigten Spannhebel, der außerhalb des Gehäuses positioniert ist und eine einhändige Bedienung ermöglicht, während das Gehäuse gehalten wird. Ein Kraftübertragungsmechanismus ist zwischen dem Spannhebel und den Trägern gekoppelt und so konfiguriert, dass die Kraft, die zum manuellen Niederdrücken des Spannhebels erforderlich ist, um die Federn in die gespannten Positionen zu zwingen, nicht zunimmt, wenn die Federn zusammengedrückt werden.

Es ist daher Aufgabe der vorliegenden Erfindung, eine verbesserte Biopsiepistole mit geringerer Länge bereitzustellen.

Erfindungsgemäß wird diese Aufgabe mit einer Biopsiepistole mit den Merkmalen von Anspruch 1 gelöst.

Im folgende wird ein Ausführungsbeispiel der Erfindung unter Bezugnahme auf die folgenden Figuren beschrieben, in welchen:
Fig. 1 eine Perspektivansicht einer Biopsiepistole gemäß dem Ausführungsbeispiel ist;
Fig. 2a eine Perspektivansicht der Biopsiepistole ohne obere Gehäusehalbschale und ohne Spanngriff ist;
Fig. 2b eine Perspektivansicht eines Rahmenelements der unteren Gehäuseschale ist;
Fig. 3a eine Draufsicht der oberen Gehäusehalbschale ist, und Fig. 3b eine Unteransicht der oberen Gehäusehalbschale ist;
Fig. 4 eine Draufsicht der Biopsiepistole ohne obere Gehäusehalbschale aber mit Spanngriff ist; Fig. 4a eine Draufsicht eines Eingriffselements ist; Fig. 4b eine Perspektivansicht des Spanngriffs ist; Fig. 4c eine Perspektivansicht eines Zapfens ist;
Fig. 5 eine perspektivische Innenansicht der oberen Gehäusehalbschale ist;
Fig. 6 eine Draufsicht eines Abschnitts der Biopsiepistole ohne obere Gehäusehalbschale in dem Zustand zeigt, in dem sich der Spanngriff in der Ausgangsposition befindet;
Fig. 7 eine Draufsicht eines Abschnitts der Biopsiepistole ohne obere Gehäusehalbschale in dem Zustand zeigt, in dem der Spanngriff in einem ersten Hub geringfügig vorgerückt wurde, sodass ein Kanülenschlitten gespannt wird;
Fig. 8 eine Draufsicht eines Abschnitts der Biopsiepistole ohne obere Gehäusehalbschale in dem Zustand zeigt, in dem der Kanülenschlitten gespannt wurde und der Spanngriff auf seine Ausgangsposition zurückgekehrt ist;

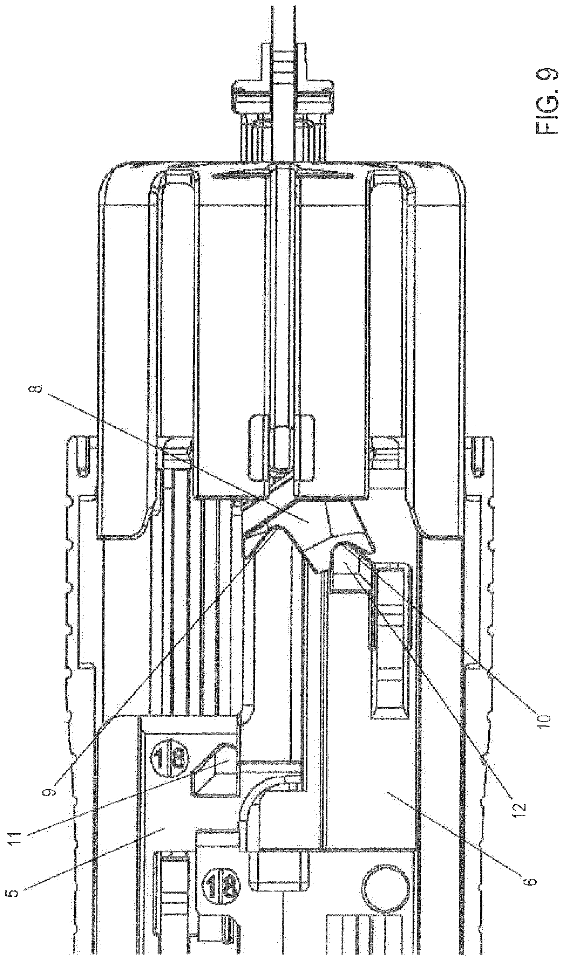

Fig. 9 eine Draufsicht eines Abschnitts der Biopsiepistole ohne obere Gehäusehalbschale in dem Zustand zeigt, in dem der Spanngriff in einem zweiten Hub geringfügig vorgerückt wurde, sodass ein Stilettschlitten gespannt wird;

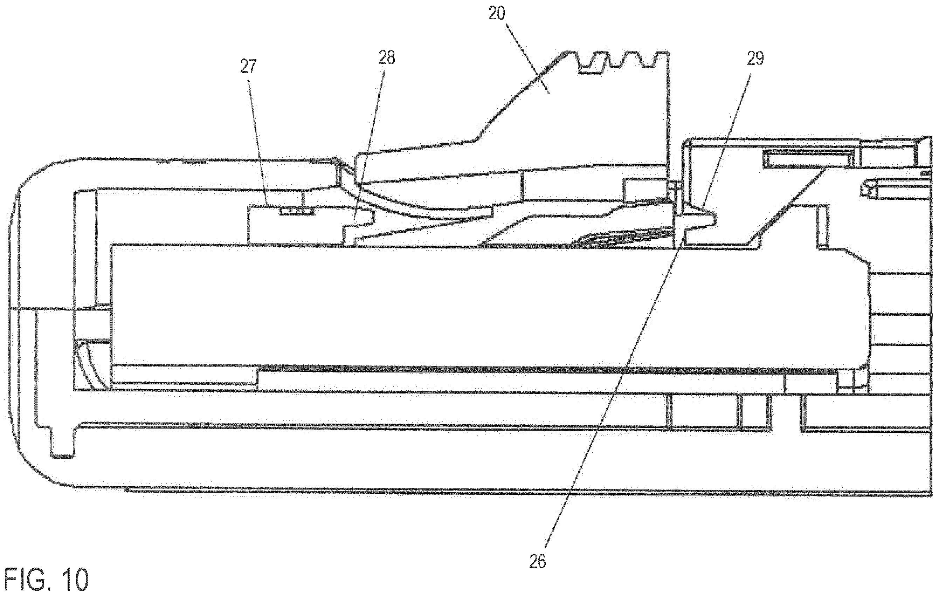

Fig. 10 einen horizontalen Längsschnitt durch die Biopsiepistole in einem Zustand zeigt, in dem der Stilettschlitten gespannt ist;
Fig. 11 einen horizontalen Längsschnitt durch die Biopsiepistole in einem Zustand zeigt, in dem der Stilettschlitten ausgelöst wurde bzw. nicht gespannt ist;

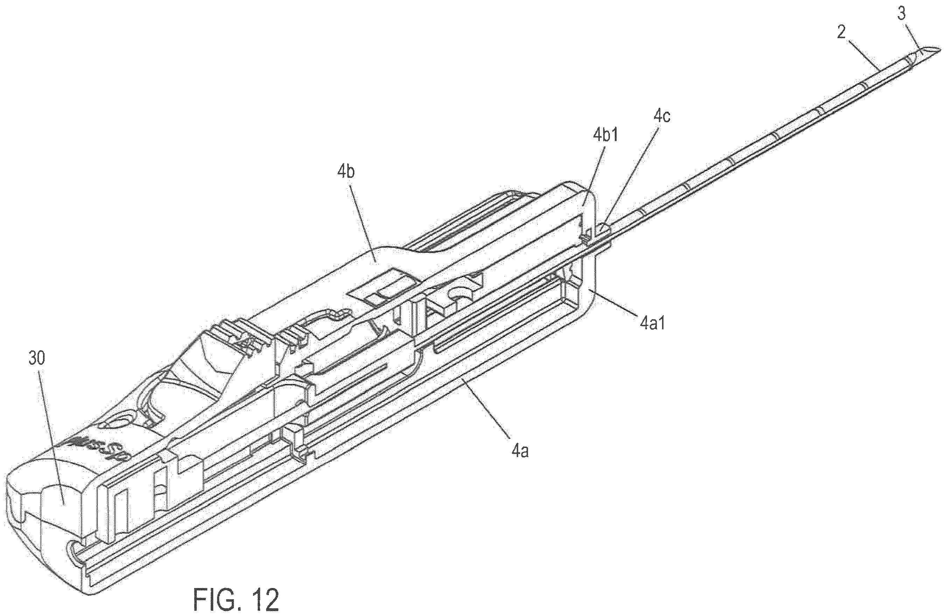

Fig. 12 einen vertikalen Längsschnitt durch die Biopsiepistole zeigt.

In der folgenden Beschreibung bezieht sich die Längsrichtung der Biopsiepistole auf die Richtung von Kanüle und Stilett, wobei die Seite mit Kanüle und Stilett die Vorderseite ist. Die Querrichtung verläuft in der Richtung, in der Kanülenschlitten und Stilettschlitten nebeneinander angeordnet sind, und die Vertikalrichtung verläuft senkrecht zu diesen beiden Richtungen, wobei die Seite mit den Tastern als die Oberseite bezeichnet wird.

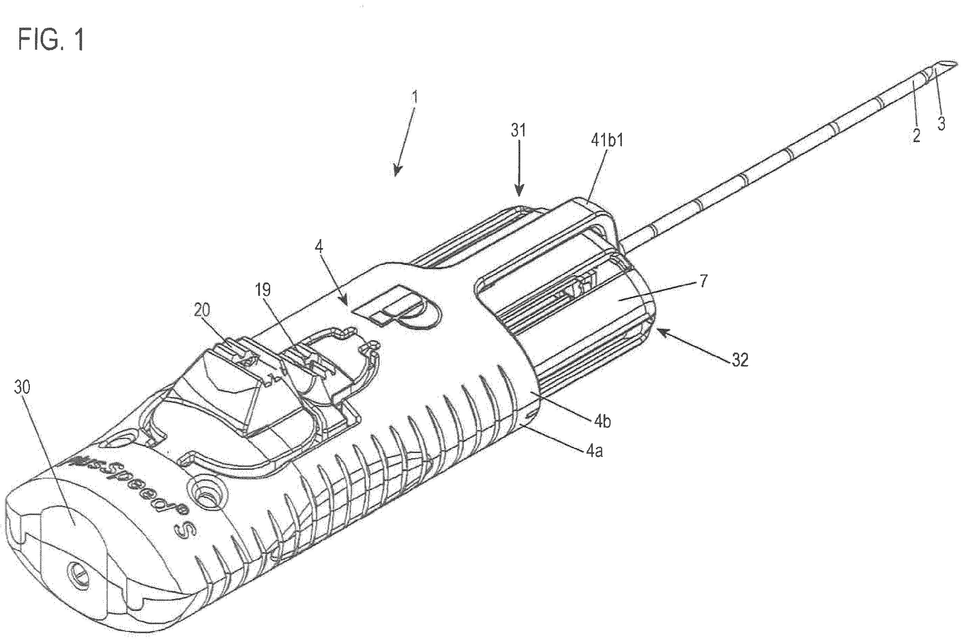

Fig. 1 zeigt eine Biopsiepistole 1 mit einem Gehäuse 4, einem Spanngriff 7, einer Kanüle 2 und einem Stilett 3. Kanüle 2 und Stilett 3 lassen sich durch zweimaliges Betätigen des Spanngriffs 7 getrennt vorspannen. Der Spanngriff 7 ist durch zwei Öffnungen 31, 32 des Gehäuses 4 für Zeigefinger und Mittelfinger einer Hand eines Anwenders zugänglich, während der Daumen des Anwenders in der dafür vorgesehenen Vertiefung 30 am anderen Ende des Gehäuses 4 abrutschsicher platziert wird. Das Gehäuse 4 besteht im Wesentlichen aus zwei Halbschalen 4a, 4b, und zwischen den Öffnungen 31, 32 des Gehäuses 4 befindet sich jeweils L-förmige Rahmenelemente 4a1, 4b1 der beiden Halbschalen 4a, 4b. Ferner sind in Fig. 1 ein erster Taster 19 zum Auslösen des Stiletts und ein zweiter Taster 20 zum Auslösen der Kanüle gezeigt.

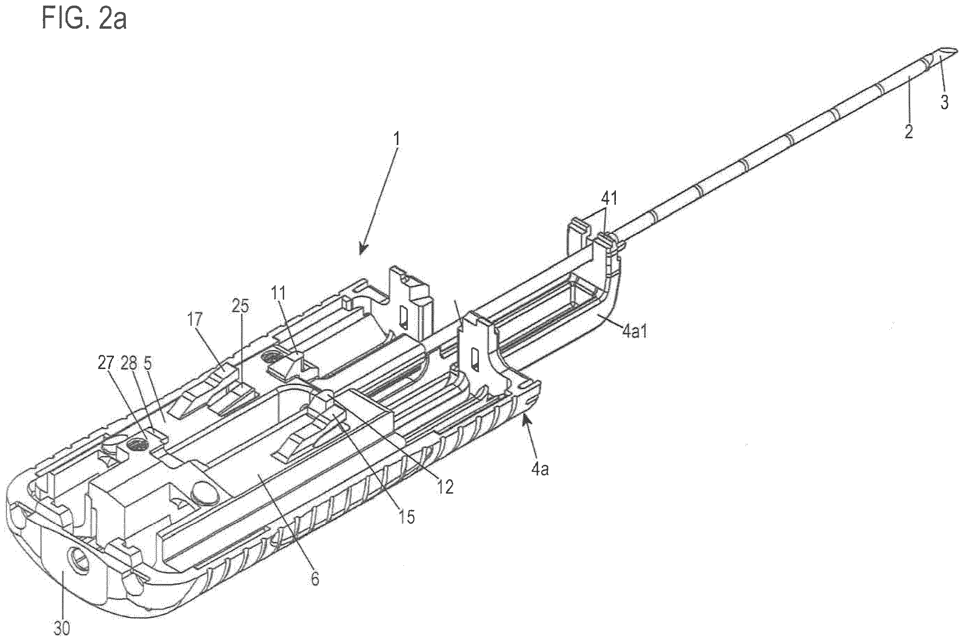

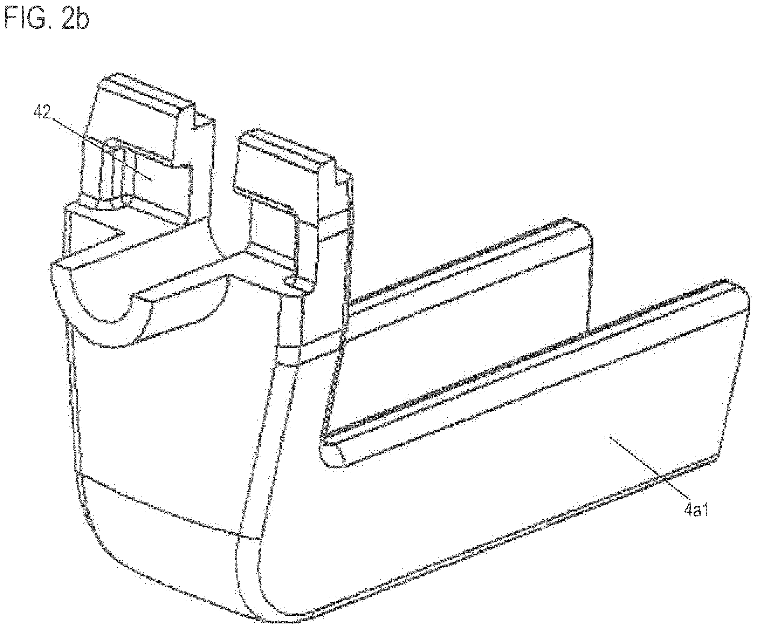

Fig. 2 zeigt die Biopsiepistole 1 ohne Spanngriff 7 und ohne obere Gehäusehalbschale 4b. Ferner hat die Biopsiepistole 1 einen Kanülenschlitten 5, der mit der Kanüle 2 verbunden ist, und einen Stilettschlitten 6, der mit dem Stilett 3 verbunden ist. Beide Schlitten können Spritzbauteile sein, die direkt an Kanüle bzw. Stilett angespritzt sind. Die Schlitten sind auf jeweiligen Schienen (nicht gezeigt) geführt und sind durch jeweilige Schraubenfedern (nicht gezeigt) nach vorne vorgespannt.

Der Kanülenschlitten 5 hat einen kanülenseitigen Vorsprung 11, der von der oberen Fläche des Kanülenschlittens 5 nach oben vorragt. In diesem Ausführungsbeispiel ist der kanülenseitige Vorsprung 11 an seiner Vorderseite abgerundet und er hat nach hinten und außen stabilisierende Schrägflächen.

Weiter hinten hat der Kanülenschlitten 5 an seiner in Querrichtung äußeren Seiten einen ersten Kanülenschnapphaken 17 und an seiner in Querrichtung inneren Seiten einen zweiten Kanülenschnapphaken 25. Beide Kanülenschnapphaken 17, 25 sind in Vertikalrichtung nachgiebig befestigt, sodass sie in Vertikalrichtung heruntergedrückt werden können. Diese nachgiebige Verbindung entsteht dadurch, dass die Kanülenschnapphaken 17, 25 auskragend und einstückig an dem Kanülenschlitten 5 ausgebildet sind.

Der Stilettschlitten 6 hat einen stilettseitigen Vorsprung 12, der von der oberen Fläche des Kanülenschlittens 6 nach oben vorragt. In diesem Ausführungsbeispiel ist der stilettseitige Vorsprung 12 an seiner Vorderseite abgerundet und er hat nach hinten und außen stabilisierende Schrägflächen.

Weiter hinten hat der Stilettschlitten 6 einen Stilettschnapphaken 15, der in Vertikalrichtung nachgiebig befestigt ist, sodass er in Vertikalrichtung heruntergedrückt werden kann. Diese nachgiebige Verbindung entsteht dadurch, dass der Stilettschnapphaken 15 auskragend und einstückig an dem Stilettschlitten 6 ausgebildet ist.

Außerdem hat der Stilettschlitten 6 ein Auslöseelement 27 mit einer nach vorne vorragenden Nase 28, die sich mit Bezug auf die Querrichtung auf einer gemeinsamen Längsachse mit dem zweiten Kanülenschnapphaken 25 befindet.

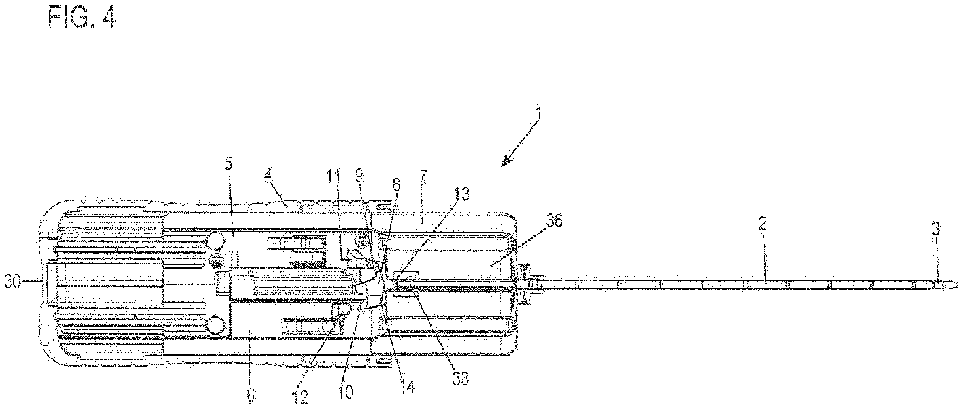

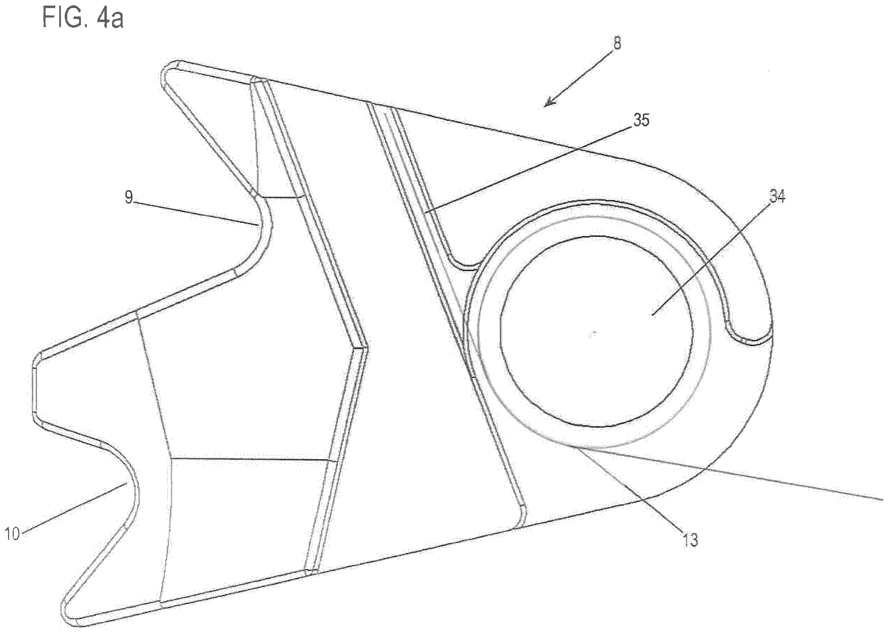

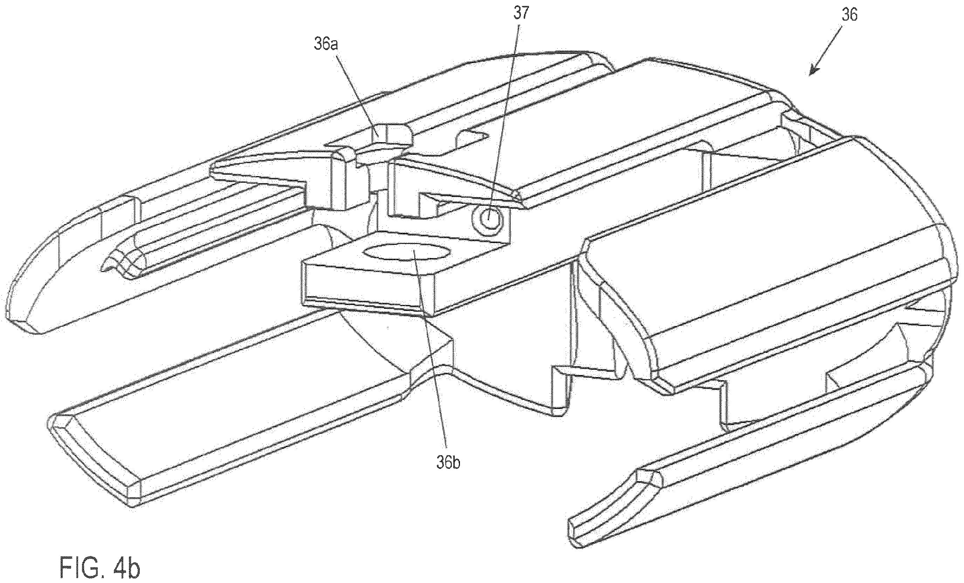

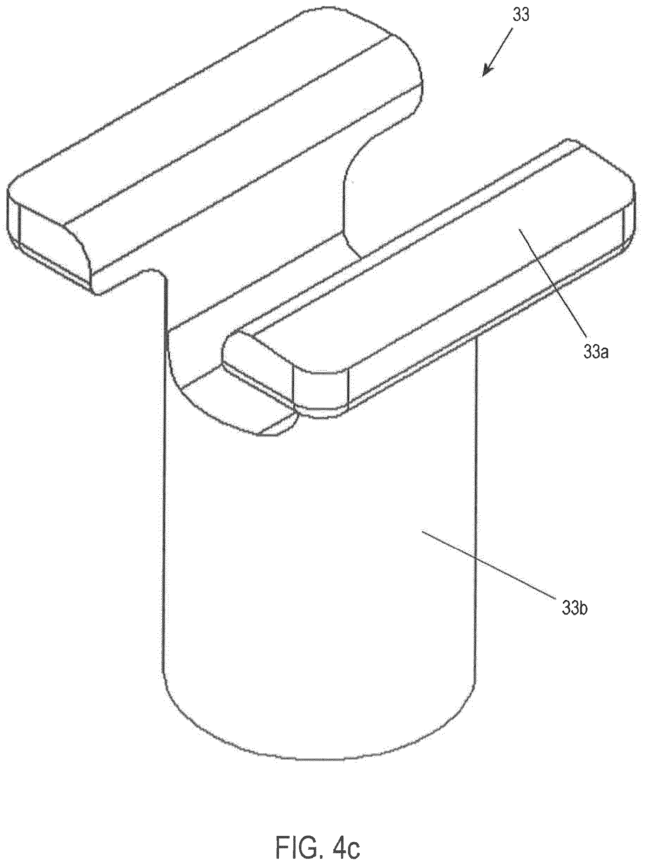

Fig. 4 zeigt die Biopsiepistole mit Spanngriff 7, an dem ein Eingriffselement 8 drehbar angebracht ist. Wie in Fig. 4a zu sehen ist, hat das Eingriffselement 8 in der Draufsicht eine im Wesentlichen dreiecksartige Form, deren vordere Spitze ein Durchgangsloch 34 hat und deren hinteren Spitzen eine kanülenseitige Vertiefung 9 und eine stilettseitige Vertiefung 10 hat. Ferner ist das Eingriffselement 8 im Bereich der Vertiefungen 9 und 10 stärker als im Bereich des Durchgangslochs 34. Zudem befindet sich im weniger starken Bereich um das Eingriffsloch 34 herum eine Federaufnahmenut 35, in der ein erstes Ende einer Schraubenfeder 13 untergebracht werden kann, während die Schraubenfeder 13 das Durchgangsloch 34 umgibt.

Fig. 4b zeigt einen Abschnitt 36 des Spanngriffs 7, der das Eingriffselement 8 drehbar trägt. Zu diesem Zweck hat der Abschnitt 36 eine rechteckige Aufnahme 36a in einem oberen Teil des Abschnitts 36 und ein Durchgansloch 36b in einem unteren Teil des Abschnitts 36. Zudem ist in dem Abschnitt 36 ein sich in Längsrichtung erstreckendes Federaufnahmeloch 37 ausgebildet, das sich in Vertikalrichtung zwischen dem oberen Teil und dem unteren Teil des Abschnitts 36 befindet und in welches ein zweites Federende eingeführt werden kann.

In Fig. 4c ist ein Zapfen 33 dargestellt, der einen zylindrischen unteren Abschnitt 33b und einen oberen Abschnitt 33a mit im Wesentlichen rechteckiger Kontur hat.

Das Eingriffselement 8 ist derart an dem Spanngriff 7 montiert, dass die Feder 13 das Durchgangsloch 34 umgibt und das erste Federende in der Federaufnahmenut 35 untergebracht ist. Ferner ist das zweite Federende in das Federaufnahmeloch 37 eingeführt und der zylindrische Abschnitt 33b des Zapfens 33 durchdringt das Durchgangsloch 34 des Eingriffselements 8 sowie das Durchgangsloch 36b des Abschnitts 36 während der obere Abschnitt 33b mit rechteckigen Kontur in der rechteckigen Aufnahme 36a aufgenommen ist. Dadurch ist das Eingriffselement 8 an dem Abschnitt 36 des Spanngriffs 7 drehbar gelagert, wobei das Eingriffselement 8 durch die Schraubenfeder 13 zunächst in Richtung des Stilettschlittens 6 vorgespannt ist.

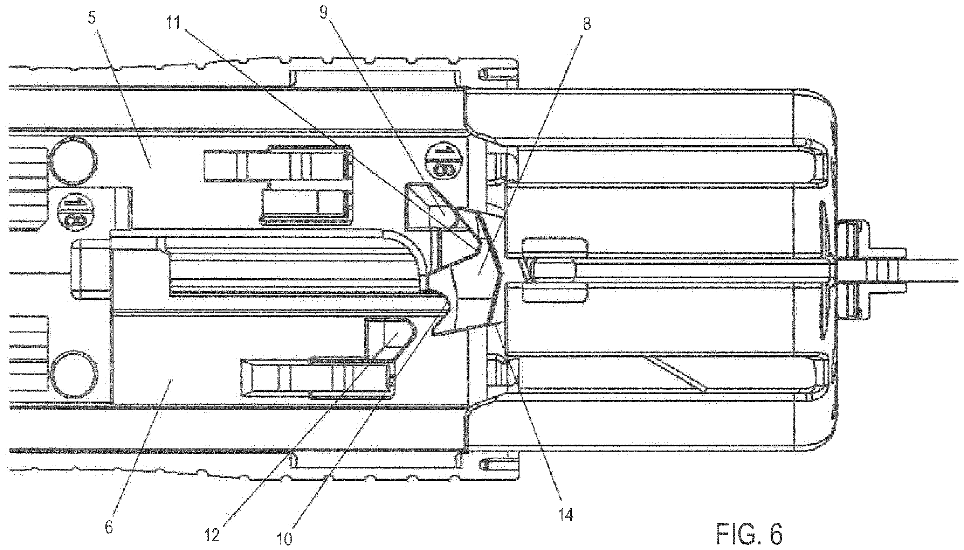

Ferner ist der Spanngriff 7 mit dem daran montierten Eingriffselement 8 derart an dem Gehäuse 4 montiert, dass das Eingriffselement 8 mit einer Anlagefläche 14 in Anlage kommt, wenn der Spanngriff durch eine Schraubenfeder (nicht gezeigt) in die Ausgangslage gespannt ist. Durch die Anlage des Eingriffselements 8 an der Anlagefläche 14 wird das Eingriffselement 8 in der Ausgangslage des Spanngriffs 7 gegen die Kraft der Feder 13 von dem Stilettschlitten weg und in Richtung zu dem Kanülenschlitten hin gedreht. Diese Position ist vergrößert in Fig. 6 dargestellt.

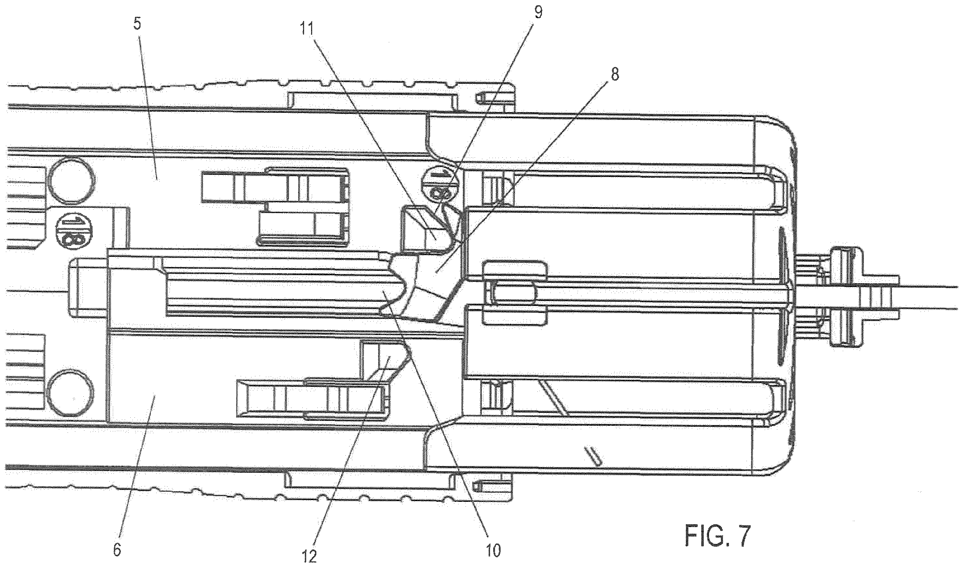

Wie in Fig. 6 zu sehen ist, ist der äußere Randbereich der Vertiefung 9 des Eingriffselements 8 in der Ausgangslage des Spanngriffs 7 mit dem kanülenseitigen Vorsprung 11 derart in Kontakt, dass das Eingriffselement 8 durch eine Betätigung des Spanngriffs 7 aus der Ausgangslage heraus gegen die Kraft der Feder 13 weiter zu dem Kanülenschlitten 5 gedreht wird. Dadurch kommt die Vertiefung 9 mit dem kanülenseitigen Vorsprung 11 sicher in Eingriff (siehe Fig. 7), sodass der Kanülenschlitten 5 durch Bewegen des Spanngriffs 7 mitbewegt wird.

Wie zudem in Fig. 7 gezeigt ist, ermöglicht die Verdrehung des Eingriffselements 8 in Richtung Kanülenschlitten, dass das Eingriffselement 8 sicher an dem stilettseitigen Vorsprung 12 vorbei geführt werden kann, ohne damit in Eingriff zu gelangen. Damit wird sichergestellt, dass bei diesem ersten Hub lediglich der Kanülenschlitten 5 gespannt wird.

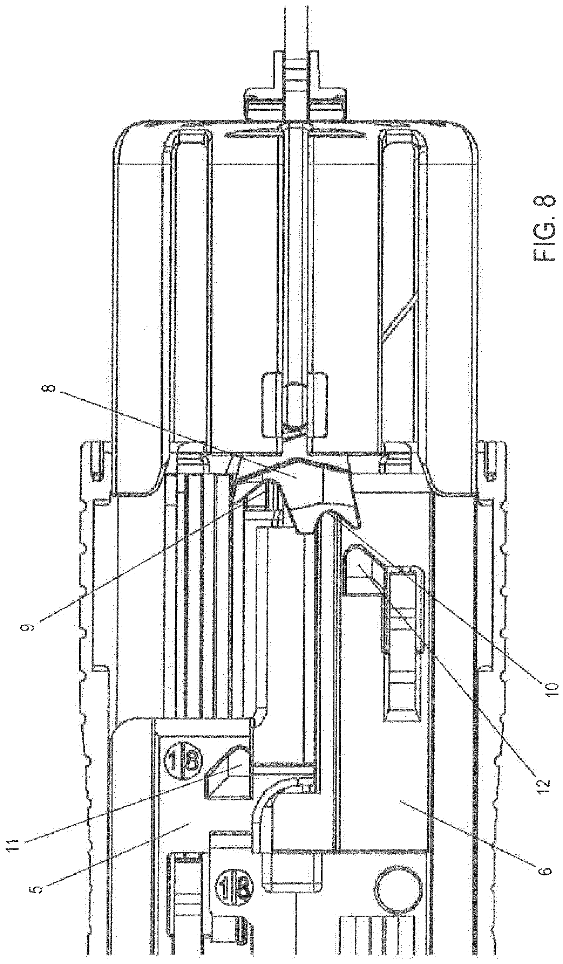

Nachdem der Kanülenschlitten 5 gespannt und mit dem Gehäuse 4 verrastet wurde (wird später genauer beschrieben), kehrt der Spanngriff 7 auf seine Ausgangslage zurück, die in Fig. 8 gezeigt ist. Da nun der Kanülenschlitten 5 gespannt ist, kommt die Vertiefung 9 nicht mehr mit dem kanülenseitigen Vorsprung 11 in Eingriff, sodass bei einer erneuten Spannbewegung des Spanngriffs 7 das Eingriffselement 8 mit dem Verlassen der Anlagefläche 14 durch die Kraft der Feder 13 in Richtung des Stilettschlittens 6 gedreht wird. Daher kann nun die Vertiefung 10 des Eingriffselement 8 mit dem stilettseitigen Vorsprung 12 in Eingriff gelangen, sodass der Stilettschlitten 6 mit der Bewegung des Spanngriffs 7 gespannt und mit dem Gehäuse 4 verrastet werden kann.

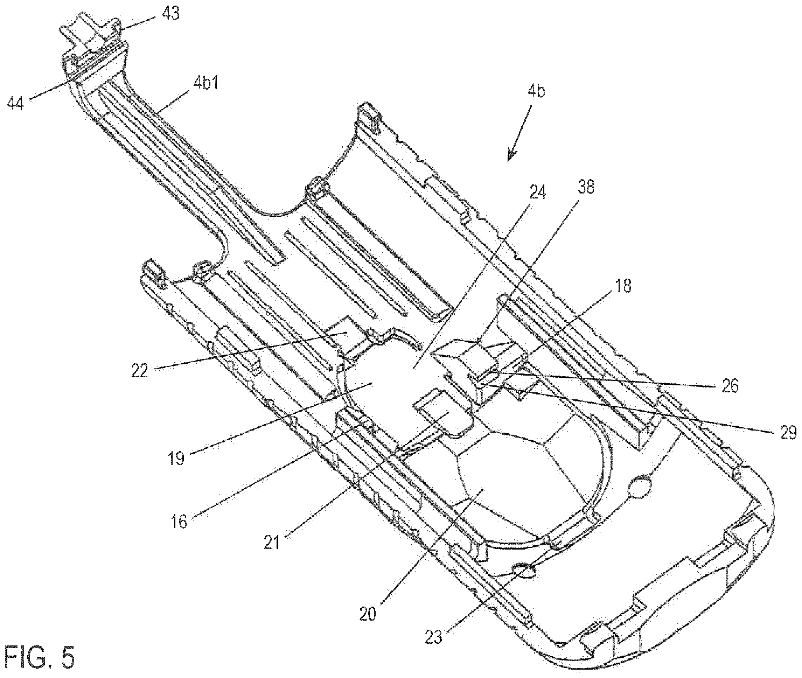

Nun wird das Einrasten der gespannten Schlitten an der Gehäuseoberschale 4b beschrieben. Wie in der Fig. 5 zu sehen ist, hat die Innenseite der oberen Gehäusehalbschale 4b in Querrichtung neben dem ersten Taster 19 zwei nach unten vorragende Bauteile, die in ihrem Sockelbereich einstückig ausgebildet sind. Das in Querrichtung außen liegende Bauteil bildet eine erste Kanülenschlittenhaltefläche 18 für den ersten Kanülenschnapphaken 17 (siehe Fig. 2a). Das in Querrichtung innen liegende Bauteil bildet eine zweite Kanülenschlittenhaltefläche 26 für den zweiten Kanülenschnapphaken 25 (siehe Fig. 2a). Beide Halteflächen 18 und 26 sind zur Rückseite gerichtet und verlaufen im Wesentlichen in Vertikalrichtung. Ferner haben beide Bauteile zur Vorderseite abgeschrägte Flächen, entlang denen die Kanülenschnapphaken 17, 25 beim jeweiligen Spannen gleiten und somit nach unten gedrückt werden, bis deren Vorderseiten hinter die Halteflächen 18, 26 gelangen und an diesen einrasten.

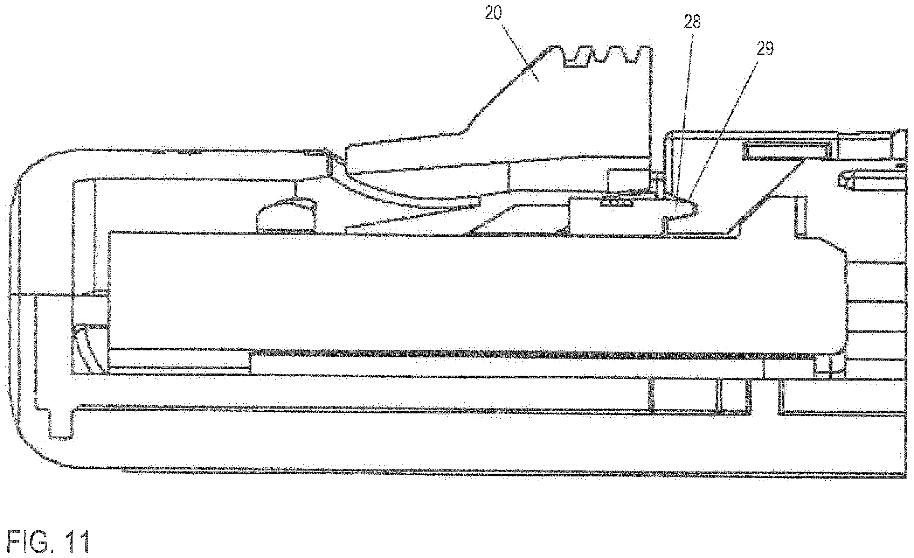

Außerdem hat das Bauteil, das die zweite Kanülenschlittenhaltefläche 26 bildet, eine Vertiefung 29, die über der zweiten Kanülenschlittenhaltefläche 26 ausgebildet ist und in Richtung der Vorderseite vertieft ist. In diese Vertiefung 29 ist die Nase 28 des Auslöseelement 27 eingetaucht, wenn sich der Kanülenschlitten 5 in seiner nicht gespannten Ausgangsposition befindet (siehe Fig. 11).

Wie ebenso in der Fig. 5 zu sehen ist, hat die Innenseite der oberen Gehäusehalbschale 4b in Querrichtung neben dem ersten Taster 19 an der den Kanülenschlittenhalteflächen 18, 26 entgegengesetzten Seite ein nach unten vorragendes Bauteil, das eine Stilettschlittenhaltefläche 16 für den Stilettschnapphaken 15 (siehe Fig. 2) bildet. Die Haltefläche 16 ist zur Rückseite gerichtet und verläuft im Wesentlichen in Vertikalrichtung. Ferner hat das vorragende Bauteil, das die Stilettschlittenhaltefläche 16 bildet, eine zur Vorderseite abgeschrägte Fläche entlang der der Stilettschnapphaken 15 beim Spannen gleitet und somit nach unten gedrückt wird, bis dessen Vorderseiten hinter die Haltefläche 16 gelangt und an dieser einrastet.

Die gespannte Biopsiepistole 1 kann auf zwei Arten ausgelöst werden. Gemäß einer ersten Art wird zuerst der erste Taster 19 betätigt, sodass nur das Stilett 3 ausgelöst wird, und danach wird der zweite Taster 20 betätigt, um die Kanüle 2 auszulösen. Gemäß einer zweiten Art wird gleich der zweite Taster 20 betätigt, wodurch erst das Stilett 3 ausgelöst wird und danach, wenn sich das Stilett vollständig nach vorne bewegt hat, die Kanüle 2 ausgelöst wird.

Nun wird die erste Art des Auslösens beschrieben.

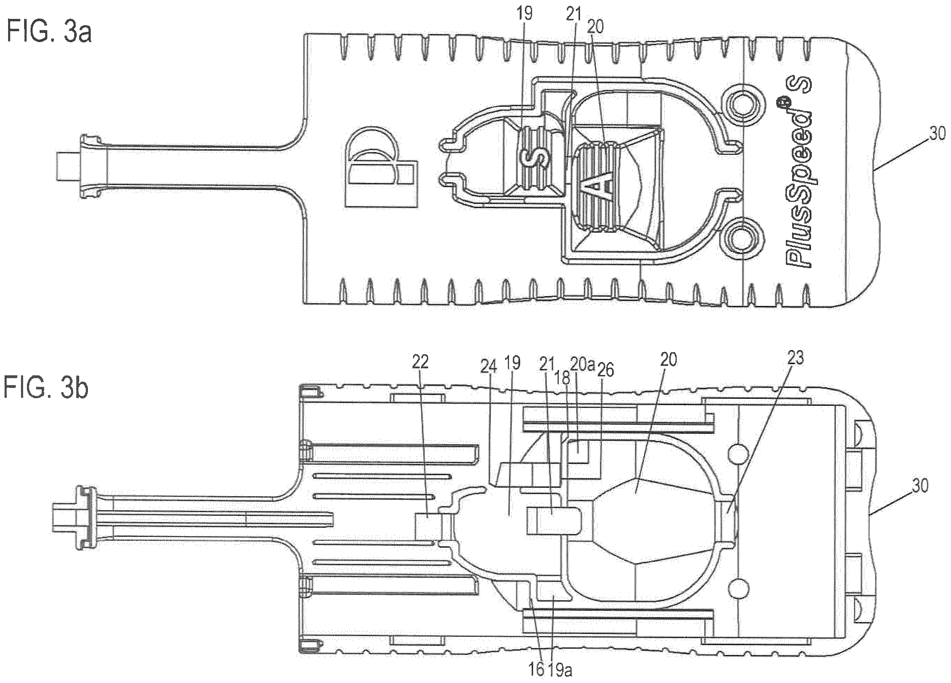

Wie in Figuren 3B und 5 zu sehen ist, ist der erste Taster 19 über ein Hauptverbindungselement 22 elastisch an dem Gehäuse 4 befestigt. Das Hauptverbindungselement 22 liegt an der Vorderseite des ersten Tasters 19, es verbindet den ersten Taster 19 einstückig mit dem Gehäuse 4 und es hat eine Querschnittsschwächung, die für die Elastizität sorgt.

Ferner ist der erste Taster 19 über ein zusätzliches Verbindungselement 24 einstückig mit dem Gehäuse 4 verbunden. Das zusätzliche Verbindungselement 24 liegt in etwa im Bereich der Bauteile, die die Halteflächen 18 und 26 bereitstellen, und damit in etwa in der auf die Längsrichtung bezogenen Mitte des ersten Tasters 19.

An der dem zusätzlichen Verbindungselement 24 in Querrichtung entgegengesetzten Seite des ersten Tasters 19 steht ein Auslösevorsprung 19a nach unten vor. Dieser Auslösevorsprung 19a wirkt im gespannten Zustand des Stilettschlittens 6 auf den Stilettschnapphaken 15, sodass bei Betätigung des ersten Tasters 19 die Vorderseite des Stilettschnapphakens 15 von der Stilettschlittenhaltefläche 16 gelöst wird und der Stilettschlitten 6 durch die Kraft der Schraubenfeder nach vorne bewegt wird.

In dem Zustand, in dem der Stilettschlitten 6 bereits nach vorne bewegt ist, der Kanülenschlitten 5 sich jedoch noch in seiner gespannten Position befindet, hat sich das Auslöseelement 27 des Stilettschlittens 6 über den ersten Kanülenschnapphaken 25 des Kanülenschlitten 5 bewegt und diesen nach unten gedrückt, sodass der Eingriff des Kanülenschnapphakens 25 an der Kanülenschlittenhaltefläche 26 gelöst wurde. Dadurch kann der Kanülenschlitten 5 erst dann ausgelöst werden, wenn das Stilett vollständig betätigt wurde.

Außerdem sorgt das zusätzliche Verbindungselement 24 dafür, dass der erste Taster 19 beim Betätigen um eine schräge Achse kippt, die im Wesentlichen durch das Hauptverbindungselement 22 und das zusätzliche Verbindungselement 24 verläuft, sodass der Auslösevorsprung 19a sicher nach unten gedrückt wird.

Wie in Figuren 3B und 5 zu sehen ist, ist der zweite Taster 20 über ein Hauptverbindungselement 23 elastisch an dem Gehäuse 4 befestigt. Das Hauptverbindungselement 23 liegt an der Hinterseite des zweiten Tasters 20, es verbindet den zweiten Taster 20 einstückig mit dem Gehäuse 4 und hat eine Querschnittsschwächung, die für die Elastizität sorgt.

An der dem Auslösevorsprung 19a des ersten Tasters 19 in Querrichtung entgegengesetzten Seite steht ein Auslösevorsprung 20a von dem zweiten Taster 20 nach unten vor. Dieser Auslösevorsprung 20a wirkt im gespannten Zustand des Kanülenschlittens 5 auf den Kanülenschnapphaken 17, sodass bei Betätigung des zweiten Tasters 20 die Vorderseite des Kanülenschnapphakens 17 von der Kanülenschlittenhaltefläche 18 gelöst wird und der Kanülenschlitten 5 durch die Kraft der Schraubenfeder nach vorne bewegt wird.

Nun wird die zweite Art des Auslösens beschrieben.

Wenn in dem Zustand, in dem beide Schlitten 5, 6 gespannt sind, der zweite Taster 20 betätigt wird, dann wirkt die Vorderseite des zweiten Tasters 20 auf eine Betätigungsnase 21 des ersten Tasters 19 ein, die sich unter den zweiten Taster 20 erstreckt (siehe Figuren 3B und 5). Dadurch wird der erste Taster 19 beim Betätigen des zweiten Tasters 20 mitbetätigt, sodass zuerst der Stilettschlitten 6 ausgelöst wird und sich nach vorne bewegt. Während dieser Bewegung ist der Kanülenschlitten jedoch noch durch den zweiten Kanülenschnapphaken 25 gehalten, der an der Kanülenschlittenhaltefläche 26 anliegt und durch den zweiten Taster 20 nicht betätigt werden kann. Gleichzeitig wird der erste Kanülenschnapphaken 17 jedoch durch die Betätigung des zweiten Tasters 20 von der ersten Kanülenschlittenhaltefläche 18 gelöst, sodass Kanülenschlitten 5 nur noch von dem zweiten Kanülenschnapphaken 25 gehalten wird.

Wenn sich nun der Stilettschlitten 6 weiter nach vorne bewegt, dann gelangt das Auslöseelement 27 über den zweiten Kanülenschnapphaken 25 und drückt diesen nach unten, sodass er von der zweiten Kanülenschlittenhaltefläche 26 gelöst wird, wenn der Stilettschlitten 6 an seiner entspannten Ausgangsposition angekommen ist. Somit wird der Kanülenschlitten 5 automatisch ausgelöst, wenn sich der Stilettschlitten entsprechend weit nach vorne bewegt hat.

Wie bei der ersten Art taucht auch hier die Nase 28 in die Vertiefung 29 ein. Der Grund dafür ist folgender.

Die Biopsiepistole 1 kann als Einweggerät ausgelegt werden, wobei die wesentlichen Bauteil Spritzgussbauteile sind, die so leicht und materialsparend wie möglich ausgebildet sind. Gleichzeitig können im Gebrauch der Biopsiepistole bspw. bei einer Prostatabiopsie große Belastungen aufgebracht werden, die dazu führen können, dass die Bestandteile sich verbiegen. Außerdem muss der zweite Kanülenschnapphaken 25 so ausgelegt sein, dass er einerseits sicher von der Schrägfläche am Bauteil, das die zweite Kanülenschlittenhaltefläche 26 aufweist, verformt wird, um danach an der zweiten Kanülenschlittenhaltefläche 26 einzurasten, andererseits muss er sicher von dem Auslöseelement 27 betätigt, also nach unten gebogen werden können. Je nach den Steifigkeits- und Belastungsverhältnissen kann jedoch der Fall eintreten, dass nicht der Kanülenschnapphaken 25 von dem Auslöseelement 27 nach unten gedrückt wird, sondern dass Kanülenschnapphaken 25 das Auslöseelement 27 nach oben drückt und somit verhindert wird, dass der Kanülenschnapphaken 25 von der Kanülenschlittenhaltefläche 26 gelöst wird. Dies würde dazu führen, dass der Kanülenschlitten nicht ausgelöst wird.

Durch das Eintauchen der Nase 28 am Auslöseelement 27 in die Vertiefung 29, die Teil der oberen Gehäusehalbschale ist, wird sichergestellt, dass der Stilettschlitten 6, insbesondere der Abschnitt, der das Auslöseelement 27 aufweist, mit Bezug auf die Vertikalrichtung in seiner Lage gehalten wird. Dadurch kann verhindert werden, dass dieser Abschnitt nach oben ausweicht, sodass der Kanülenschnapphaken 25 stets sicher gelöst wird.

Wie bereits erläutert wurde, besteht das Gehäuse 4 aus zwei Gehäusehalbschalen 4a und 4b mit L-förmigen Rahmenelementen 4a1 und 4b1, an deren vorderen Enden jeweils ein halbzylinderförmiger Abschnitt ausgebildet ist, die zusammen einen Stummelkegel 4c bilden, der als ein Kanülenführungsabschnitt dient. An dieser Stelle ist das Gehäuse besonders starken Belastungen ausgesetzt, da sich die Kanüle 2 und das Stilett 3 bspw. bei einer Prostatabiopsie stark verbiegen können und somit seitliche Kräfte auf den Stummelkegel 4c aufbringen, die auf die Innenseite des Stummelkegels 4c radial auswärts wirken. Ohne weiter Maßnahmen besteht daher die Gefahr, dass sich die Gehäusehalbschalen hier voneinander lösen, sodass eine einwandfreie Handhabung des Geräts nicht mehr gewährleistet ist.

Daher sind die beiden Rahmenelement 4a1 und 4b1 im Bereich des Stummelkegels 4c in beiden zueinander entgegengesetzten Längsrichtungen miteinander verrastet. Dazu hat das L- förmige Rahmenelement 4a1 an dem innenliegenden Ende seines kurzen Schenkels Vorsprünge 41 an beiden Seiten einer Aussparung, durch die die Kanüle 2 geführt ist (siehe Fig. 2a). An der vorderen Seite des kurzen Schenkels (siehe Fig. 2b) ist die eine Hälfte des Kegelstummels 4c ausgebildet, wobei sich zwei aufgegabelte Abschnitte des kurzen Schenkels mit nach hinten abgesenkten Niveau weiter Richtung Rahmenelement 4b1 erstrecken. In jedem aufgegabelten Abschnitt ist an der Vorderseite eine zur Innenseite zwischen den aufgegabelten Abschnitten offene Vertiefung 42 ausgebildet.

Fig. 5 zeigt die Gestaltung des kurzen Schenkels des L-förmigen Rahmenelements 4b1. Hier ist die zweite Hälfte des Kegelstummels 4c am Ende des kurzen Schenkels ausgebildet. Die nach hinten zeigende Fläche dieses Endes hat einen Vorsprung 43, der in die beiden Vertiefungen 42 des Rahmenelements 4a1 passt. Durch Zusammenwirken von Vorsprung 43 und Vertiefungen 42 sind die Enden der kurzen Schenkel der Rahmenelemente 4a1 und 4b1 in allen Richtungen senkrecht zur Längsachse aneinandergehalten.

Außerdem hat das Ende des kurzen Schenkels des Rahmens 4b1 einen Vorsprung 44, der sich an der hinteren Seite dieses Endes in Richtung des Rahmenelements 4a1 erstreckt und mit dem Vorsprung 41 des Rahmenelements 4a1 in Anlage gebracht werden kann. Somit sind die beiden Rahmenelemente 4a1 und 4b1 an deren Enden in der ersten Längsrichtung der Biopsiepistole 1 verrastet.

Ferner liegt der Bereich des Endes des kurzen Schenkels des Rahmenelements 4b1 vor den aufgegabelten Abschnitten des Endes des kurzen Schenkels des Rahmenelements 4a1. Somit sind die beiden Rahmenelemente 4a1 und 4b1 an deren Enden in der zweiten Längsrichtung, die der ersten Längsrichtung entgegengesetzt ist, verrastet.

Somit kann der Kegelstummel 4c im montierten Zustand starken Belastungen durch die gebogene Kanüle ausgesetzt werden, ohne dass sich die Rastverbindung ungewollt löst.

Die Gehäusehalbschalen 4a, 4b haben zudem weitere Verrastungselemente, die die Gehäusehalbschalen 4a, 4b in Längsrichtung und Querrichtung zuverlässig miteinander verbindet.

## Patentansprüche

1. Biopsiepistole (1) mit einer Kanüle (2) und einem in der Kanüle (2) geführten Stilett (3), wobei die Biopsiepistole (1) ferner folgendes aufweist:
ein Gehäuse (4),
einen Kanülenschlitten (5) zum Bewegen der Kanüle (2),
einen Stilettschlitten (6) zum Bewegen des Stiletts (3),
einen Spanngriff (7) zum Spannen des Kanülenschlittens (5) und des Stilettschlittens (6) von einer Ausgangsposition in eine Spannposition,
ein Eingriffselement (8), das an dem Spanngriff (7) vorgesehen ist, und Vertiefungen (9, 10) hat, die mit entsprechenden Vorsprüngen (11, 12) des Kanülenschlittens (5) und des Stilettschlittens (6) in Eingriff gelangen können, wobei
das Eingriffselement (8) an dem Spanngriff (7) drehbar angebracht ist und mittels einer Feder (13) gegen eine Anlagefläche (14) des Gehäuses (4) vorgespannt ist,
der Spanngriff (7) einen Abschnitt (36) aufweist, der das Eingriffselement (8) drehbar trägt und ein Federaufnahmeloch (37) aufweist,
das Eingriffselement (8) eine Federaufnahmenut (35) aufweist,
ein erstes Ende der Feder (13) in die Federaufnahmenut (35) eingesetzt ist und ein zweites Ende der Feder (13) in das Federaufnahmeloch (37) eingesetzt ist, wobei
das Eingriffselement (8) eine kanülenseitige Vertiefung (9) und eine stilettseitige Vertiefung (10) hat, der Kanülenschlitten (5) einen kanülenseitigen Vorsprung (11) hat und der Stilettschlitten (6) einen stilettseitigen Vorsprung (12) hat, wobei
der kanülenseitige Vorsprung (11) zumindest in der Ausgangsposition dem Eingriffselement (8) näher als der stilettseitige Vorsprung (12) ist, wobei
das Eingriffselement (8), die Anlagefläche (14), der kanülenseitige Vorsprung (11) und der stilettseitige Vorsprung (12) so ausgelegt sind, dass das Eingriffselement (8) in der nicht betätigten Position des Spanngriffs (7) durch die Feder (13) und die Anlagefläche (14) in einer solchen Drehposition gehalten ist, dass die kanülenseitige Vertiefung (9) mit dem kanülenseitigen Vorsprung (11) in Eingriff gelangt, sodass bei einer Betätigung des Spanngriffs (7) das Eingriffselement (8) durch den Eingriff zwischen der kanülenseitigen Vertiefung (9) und dem kanülenseitigen Vorsprung (11) gegen die Federkraft zu der Seite des kanülenseitigen Vorsprungs (11) gedreht wird, sodass die stilettseitige Vertiefung (10) beim Spannen des Kanülenschlittens (5) mit dem stilettseitigen Vorsprung (12) nicht in Eingriff gelangen kann.

2. Biopsiepistole (1) gemäß Anspruch 1, wobei
die Anlagefläche (14) des Gehäuses (4) an der Seite des Eingriffselements (8) liegt, die dem Stilettschlitten (6) entspricht.

## Claims

1. A biopsy gun (1) having a cannula (2) and a stylet (3) guided in the cannula (2), the biopsy gun (1) further comprising:
a housing (4),
a cannula carriage (5) for moving the cannula (2),
a stylet carriage (6) for moving the stylet (3),
a tensioning grip (7) for tensioning the cannula carriage (5) and the stylet carriage (6) from an initial position to a tensioning position,
an engagement element (8) which is provided on the tensioning grip (7) and has recesses (9, 10) which can engage with corresponding projections (11, 12) of the cannula carriage (5) and the stylet carriage (6), wherein
the engagement element (8) is rotatably mounted on the tensioning grip (7) and is pretensioned against a contact surface (14) of the housing (4) by means of a spring (13),
the tensioning grip (7) has a section (36) which rotatably supports the engagement element (8) and has a spring-receiving hole (37),
the engagement element (8) has a spring-receiving groove (35),
a first end of the spring (13) is inserted into the spring-receiving groove (35) and a second end of the spring (13) is inserted into the spring-receiving hole (37), wherein
the engagement element (8) has a cannula-side recess (9) and a stylet-side recess (10), the cannula carriage (5) has a cannula-side projection (11) and the stylet carriage (6) has a stylet-side projection (12), wherein
the cannula-side projection (11) is closer to the engagement element (8) than the stylet-side projection (12), at least in the initial position, wherein
the engagement element (8), the contact surface (14), the cannula-side projection (11) and the stylet-side projection (12) are designed such that, in the non-actuated position of the tensioning grip (7), the engagement element (8) is held by the spring (13) and the contact surface (14) in such a rotational position that the cannula-side recess (9) is engaged with the cannula-side projection (11), so that, when the tensioning grip (7) is actuated, the engagement element (8) is rotated towards the side of the cannula-side projection (11) by the engagement between the cannula-side recess (9) and the cannula-side projection (11) against the spring force, so that the stylet-side recess (10) cannot engage with the stylet-side projection (12) when the cannula carriage (5) is tensioned.

2. The biopsy gun (1) according to claim 1, wherein
the contact surface (14) of the housing (4) lies on the side of the engagement element (8) which corresponds to the stylet carriage (6).

## Revendications

1. Pistolet à biopsie (1) comprenant une canule (2) et un stylet (3) guidé dans la canule (2), le pistolet à biopsie (1) comprenant en outre :
un boîtier (4),
un chariot de canule (5) pour déplacer la canule (2),
un chariot de stylet (6) pour déplacer le stylet (3),
une poignée de serrage (7) pour serrer le chariot de canule (5) et le chariot de stylet (6) d'une position initiale à une position de serrage,
un élément d'engagement (8) prévu sur la poignée de serrage (7) et ayant des cavités (9, 10) qui peuvent s'engager avec des saillies correspondantes (11, 12) du chariot de canule (5) et du chariot de stylet (6), dans lequel
l'élément d'engagement (8) est monté de manière rotative sur la poignée de serrage (7) et est précontraint par un ressort (13) contre une surface d'appui (14) du boîtier (4),
la poignée de serrage (7) comprend une partie (36) qui supporte de manière rotative l'élément d'engagement (8) et qui comprend un trou de réception de ressort (37),
l'élément d'engagement (8) comprend une rainure de réception de ressort (35),
une première extrémité du ressort (13) est insérée dans la rainure de réception de ressort (35) et une deuxième extrémité du ressort (13) est insérée dans le trou de réception de ressort (37), dans lequel
l'élément d'engagement (8) a une cavité côté canule (9) et une cavité côté stylet (10), le chariot de canule (5) a une saillie côté canule (11) et le chariot de stylet (6) a une saillie côté stylet (12), dans lequel
la saillie côté canule (11) est plus proche de l'élément d'engagement (8) que de la saillie côté stylet (12), au moins dans la position initiale, dans lequel
l'élément d'engagement (8), la surface d'appui (14), la saillie côté canule (11) et la saillie côté stylet (12) sont conçus de telle sorte que l'élément d'engagement (8), dans la position non actionnée de la poignée de serrage (7), est maintenu par le ressort (13) et la surface d'appui (14) dans une position de rotation telle que la cavité côté canule (9) s'engage avec la saillie côté canule (11), de sorte que lorsque la poignée de serrage (7) est actionnée, l'élément d'engagement (8) est tourné vers le côté de la saillie côté canule (11) par l'engagement entre la cavité côté canule (9) et la saillie côté canule (11), à l'encontre de la force du ressort, de sorte que la cavité côté stylet (10) ne peut pas s'engager avec la saillie côté stylet (12) lorsque le chariot de canule (5) est serré.

2. Pistolet à biopsie (1) selon la revendication 1, dans lequel
la surface d'appui (14) du boîtier (4) se trouve du côté de l'élément d'engagement (8) qui correspond au chariot de stylet (6).
